# EUROPÄISCHE PATENTSCHRIFT

(11) **EP 2 248 462 B1**
(45) Veröffentlichungstag und Bekanntmachung des Hinweises auf die Patenterteilung: **20.04.2016**
(21) Anmeldenummer: 09159533.0
(22) Anmeldetag: 06.05.2009
(51) Int. Cl.: A61B 5/00, A61B 90/00, G01N 21/64, A61B 5/06

(54) **Verfahren zur Darstellung von Bilddaten eines Patientenkörperteils**
Method for portraying image data of a patient body part
Procédé de représentation de données d'image d'une partie du corps d'un patient

(43) Veröffentlichungstag der Anmeldung: 10.11.2010
(73) Patentinhaber: Brainlab AG, 85622 Feldkirchen (DE)
(72) Erfinder: Wörlein, Swen, 80637 München (DE); Weissenborn, Anke, 85622 Feldkirchen (DE); Thiemann, Ingmar, 80686 München (DE); Mathis, Martin, 85653 Aying-Grosshelfendorf (DE)
(74) Vertreter: Schwabe - Sandmair - Marx

(56) Entgegenhaltungen:
- DE-A1- 10 339 784
- US-A1- 2005 182 321
- US-A1- 2007 073 159
- US-A1- 2008 294 056

## Beschreibung

Die Erfindung betrifft ein Verfahren zur Darstellung von Bilddaten eines Patientenkörperteils und eine Vorrichtung hierzu. Insbesondere betrifft die Erfindung ein Verfahren, bei dem in einem ersten Schritt ein Bild eines mit einem fluoreszierenden Kontrastmittel versehenen Patientenkörperteil aufgenommen wird und aufgenommene Bilddaten in einem späteren zweiten Schritt einem Benutzer dargestellt werden. Die Erfindung betrifft auch eine entsprechende Vorrichtung zur Durchführung dieses Verfahrens.

Aus dem Stand der Technik sind Verfahren bekannt, bei denen bestimmte interessierende Bereiche eines Patientenkörperteils, beispielsweise Tumorgewebe mittels eines fluoreszierenden Kontrastmittels markiert werden. Da sich das Kontrastmittel besonders gut an das Tumorgewebe anlagert, leuchtet das Tumorgewebe unter bestimmten Lichtverhältnissen im Vergleich zum umliegenden gesunden Gewebe heller. Soll das Tumorgewebe chirurgisch entfernt werden, so müssen lediglich die hell leuchtenden Bereiche des Patientenkörperteils entfernt werden. Zum guten Sichtbarmachen der von den fluoreszierenden Substanzen emittierten Fluoreszenzstrahlung ist es jedoch oftmals notwendig, im Operationsraum besondere Lichtverhältnisse zu erzeugen. Soll im Anschluss daran das identifizierte Tumorgewebe entfernt werden, so muss dazu die für Operationen notwendige Operationssaalbeleuchtung eingeschaltet werden. Durch Wiederholen dieser Schritte kann auch noch am Patienten verbliebenes Tumorgewebe entfernt werden. Somit ist ein ständiges Hin- und Herschalten zwischen Operationssaalbeleuchtung und den für die Sichtbarmachung des zu entfernenden Tumorgewebes notwendigen besonderen Lichtverhältnissen notwendig. Dies stört den normalen Operationsablauf erheblich.

Auch stellt es oftmals ein Problem dar, wenn die Leuchtkraft des Kontrastmittels innerhalb kurzer Zeit rapide abnimmt oder das Kontrastmittel durch den Blutstrom ständig fortgetragen wird. In diesen Fällen muss dem Patientenkörperteil ständig neues Kontrastmittel zugeführt werden.

Aus der DE 103 39 784 A1 ist ein Mikroskopiesystem und ein entsprechendes Verfahren bekannt, bei welchem in einem der Strahlengänge des Mikroskops ein Fluoroskopiebild eingeblendet wird.

Es ist Aufgabe der Erfindung, ein Verfahren und eine Vorrichtung bereitzustellen, mit deren Hilfe Bereiche eines Patientenkörperteils mittels eines Kontrastmittels dauerhaft gut sichtbar markiert werden können, ohne den Operationsablauf unter normalen Lichtverhältnissen zu stören.

Diese Aufgabe wird durch das Verfahren gemäß dem Patentanspruch 1 bzw. die Vorrichtung gemäß dem Patentanspruch 11 gelöst.

Beim erfindungsgemäßen Verfahren wird ein Patientenkörperteil mit Bereichen, die mit Strahlung emittierendem Kontrastmittel versehen sind, in einem ersten Schritt mittels einer Erfassungseinrichtung erfasst, wobei während des ersten Schritts die Intensität der von den mit dem Kontrastmittel versetzten Bereichen emittierten Strahlung höher ist, als die Intensität anderer von der Erfassungseinrichtung erfassbarer Strahlung, die vom erfassten Patientenkörperteil ausgehen, wobei auf Basis der erfassten Strahlung ein Patientenbilddatensatz erstellt wird, und wobei der Patientenbilddatensatz in einem zweiten, insbesondere späteren Schritt einem Benutzer mittels einer Ausgabeeinrichtung dargestellt wird.

Mit anderen Worten wird also einem Patientenkörperteil ein Kontrastmittel zugeführt, welches Strahlung emittiert und somit Bereiche sichtbar macht, in denen sich das Kontrastmittel besonders gut anlagert. Sofern das Kontrastmittel Strahlung im sichtbaren Lichtspektrum emittiert, erscheinen diese Bereiche dem bloßen Auge heller als andere Bereiche, in denen sich weniger oder kein Kontrastmittel anlagert.

Ein solches Kontrastmittel kann beispielsweise ALA (Aminolevulinic Acid) für die Markierung von Tumorgewebe oder ICG (Indocyaningrün) für die Markierung von Blutgefäßen sein. Auch ist ein Kontrastmittel denkbar, welches Strahlung im Infrarotlichtbereich oder UV-Licht-Bereich emittiert.

Um die kenntlich zu machenden Bereiche mit Kontrastmittel zu versehen, ist eine ein- oder mehrmalige Injektion von Kontrastmittel vorstellbar, wobei sich das Kontrastmittel selbstständig an bestimmte interessierende Bereiche anlagert oder bevorzugt in oder an diesen vorherrscht. Auch ist eine stetige Infusion von Kontrastmittel vorstellbar. Ebenso wäre ein manuelles Auf- oder Einbringen von Kontrastmittel an bzw. in bestimmte kenntlich zu machende Bereiche vorstellbar.

Der Patientenkörperteil mit den mit Kontrastmittel versehenen Bereichen wird im Anschluss daran mittels einer Erfassungseinrichtung erfasst bzw. aufgenommen. Dies geschieht vorteilhafter Weise zu einem Zeitpunkt kurz nachdem die kenntlich zu machenden Bereiche mit dem Kontrastmittel versehen wurden, da zu diesem Zeitpunkt die Emissionsstärke des Kontrastmittels sehr hoch ist und daher sehr kontrastreiche Aufnahmen des Patientenkörperteils mit den kenntlich gemachten Bereichen möglich sind. Sofern die Emissionsstärke des Kontrastmittels über die Zeit nachlässt oder das Kontrastmittel durch fließende Körperflüssigkeiten fortgetragen wird, wird die Notwendigkeit einer möglichst kurz nach dem Versehen der kenntlich zu machenden Bereiche mit Kontrastmittel erfolgenden Erfassung durch die Erfassungseinrichtung deutlich. Zusammenfassend lässt sich also sagen, dass die Erfassung des Patientenkörperteils zu einem Zeitpunkt erfolgt, bei dem die Strahlungsintensität des Kontrastmittels höher ist als die Intensität anderer vom Patientenkörperteil ausgehender Strahlung. Kontrastreiche Aufnahmen des Patientenkörperteils mit deutlich sichtbar markierten Bereichen sind die Folge.

Auf Basis der von der Erfassungseinrichtung erfassten Bilder vom Patientenkörperteil wird ein Patientenbilddatensatz erstellt, wobei ein oder mehrere aufgenommene Bilder hinsichtlich ihrer Lage und Ausrichtung relativ zum realen Patientenkörperteil ausgerichtet werden. Auch können einzelne aufgenommene Bilder relativ zueinander ausgerichtet werden.

Der Vorteil des erfindungsgemäßen Verfahrens zeigt sich insbesondere im nun folgenden zweiten Schritt, nämlich bei der Darstellung des Patientenbilddatensatzes mittels einer Ausgabeeinrichtung. Trotz der Tatsache, dass seit dem Versehen der kenntlich gemachten Bereiche mit Kontrastmittel die Emissionsstärke bzw. Leuchtkraft des Kontrastmittels in erheblicher Weise abgenommen haben könnte, was die Identifizierung dieser Bereiche erschwert, stehen dem Benutzer durch das erfindungsgemäße Verfahren zu jedem beliebigen späteren Zeitpunkt kontrastreiche Bilder mit deutlich abgegrenzten mit Kontrastmitteln markierten Bereichen zur Verfügung. Ebenso besteht die Möglichkeit, die erstellten Bilder mittels einer Ausgabeeinrichtung bei normalen Lichtverhältnissen im Operationssaal während einer Operation darzustellen, ohne an spezielle Lichtverhältnisse wie beispielsweise einen verdunkelten Operationssaal gebunden zu sein. Kontrastreiche Bilder vom Patientenkörperteil mit den durch Kontrastmittel markierten Bereichen stehen also auch während eines hell erleuchteten Operationssaals zur Verfügung.

In einer bevorzugten Ausführungsform emittiert das Kontrastmittel Strahlung im sichtbaren Lichtspektrum. Es ist jedoch prinzipiell ebenso denkbar, dass das Kontrastmittel nicht sichtbare Strahlung wie beispielsweise Röntgenstrahlung, radioaktive Strahlung (z. B. α-, β-, γ-Strahlung), Infrarotstrahlung oder UV-Strahlung emittiert. Prinzipiell ist es auch denkbar, dass das Kontrastmittel ein magnetisches Material umfasst, welches anhand der magnetischen Eigenschaften des Materials detektierbar ist.

Es ist ferner vorstellbar, dass einzelne zu markierende Bereiche des Patientenkörperteils mit mehreren unterschiedlichen Kontrastmitteln versetzt werden, die jeweils unterschiedliche oder gleichartige Strahlung emittieren oder unterschiedliche Eigenschaften aufweisen. So können vom Patientenkörperteil mittels unterschiedlicher bildgebender Verfahren Aufnahmen erstellt werden, in denen die zu markierenden Bereiche klar von den übrigen Bereichen des Patientenkörperteils abgegrenzt sind.

In einer weiteren bevorzugten Ausführungsform umfasst der Patientenbilddatensatz die Oberflächendaten des Patientenkörperteils. Es ist grundsätzlich möglich, die zu markierenden Bereiche des Patientenkörperteils mit einem Strahlung emittierenden Kontrastmittel zu versetzen. Sofern zum Erfassen der emittierten Strahlung durch die Erfassungseinrichtung ein direkter Sichtkontakt zwischen Erfassungseinrichtung und dem Kontrastmittel bzw. den mit dem Kontrastmittel versetzten Bereichen notwendig ist, macht die Erfassung nur Sinn, sofern die zu erfassende Strahlung von Oberflächenteilen am Patientenkörperteil aus emittiert wird. Sobald die emittierte Strahlung jedoch auch durch Patientengewebe hindurch detektierbar sein soll, können auch innerhalb des Patientenkörperteils befindliche und mit Kontrastmittel versetzte Bereiche ohne direkten Sichtkontakt mittels der Erfassungseinrichtung erfasst werden. Dies wäre beispielsweise dann der Fall, wenn das Kontrastmittel Röntgenstrahlung oder radioaktive Strahlung emittiert. Auch ein magnetisches Material umfassendes Kontrastmittel innerhalb des Patientenkörperteils könnte prinzipiell durch eine außerhalb des Patientenkörperteils angeordnete Erfassungseinrichtung ohne jeglichen direkten Sichtkontakt erfasst werden.

Ferner kann der Patientenbilddatensatz eine Vielzahl von zu unterschiedlichen Zeitpunkten erfassten Bildern vom Patientenkörperteil umfassen. So ist es vorstellbar, dass eine Serie von Bildern des Patientenkörperteils aus dem gleichen Blickwinkel in einer zeitlichen Abfolge erstellt wird, so dass ein zeitlicher Verlauf in dem Patientenbilddatensatz erkennbar wird. Dieser zeitliche Verlauf kann beispielsweise Gewebebewegungen oder eine Veränderung von Ort oder Strahlungsintensität des Kontrastmittels erkennbar machen. Insbesondere kann vom Patientenkörperteil eine Videofilmaufnahme erstellt werden, welche zu einem späteren Zeitpunkt beispielsweise in einer Endlosschleife abgespielt werden kann.

In einer weiteren bevorzugten Ausführungsform werden aus zumindest zwei unterschiedlichen Blickrichtungen zeitgleich Bilder oder Filme vom Patientenkörperteil erstellt. Sofern die Position und die Ausrichtung der Erfassungseinrichtung und auch die Lage der Blickrichtungen im Raum bekannt ist, lässt sich auch die Position und Ausrichtung von auf mehreren Bildern erkennbaren und einander zuordenbaren Objekten durch Triangulation bestimmen. Letztendlich ist es möglich, auf Basis dieser Bilder einen 3D-Bilddatensatz zu erstellen, aus dem die Lage und Ausrichtung einzelner Objekte und auch der markierten Bereiche entnehmbar ist. So ist es möglich, durch ein medizintechnisches Navigationssystem im Raum ortbare Instrumente zu den markierten Bereichen zu führen.

Gemäß der Erfindung wird der Patientenbilddatensatz im zweiten Schritt mittels der Ausgabeeinrichtung dem Blickfeld eines Chirurgen überlagert. So ist es möglich, dass der Chirurg die erzeugten Bilddaten betrachten kann, ohne den Blick vom Patienten bzw. dem Patientenkörperteil abwenden zu müssen. Das Überlagern der Patientenbilddaten kann dabei beispielsweise nach Art eines Head-Up-Displays geschehen. Sobald der Chirurg also durch ein solches ein Head-Up-Display aufweisendes Gerät wie beispielsweise eine Brille oder ein Mikroskop blickt, sieht er sowohl den zu behandelnden Patienten als auch die eingeblendeten Patientenbilddaten.

Da der Patientenbilddatensatz unter Verwendung einer getrackten Erfassungseinrichtung erstellt wurde, ist sowohl die Position als auch die Ausrichtung der durch das Kontrastmittel markierten Bereiche im Raum bekannt. Wenn der Patient bzw. der Patientenkörperteil nach dem Erfassen der durch das Kontrastmittel emittierten Strahlung durch die Erfassungseinrichtung und dem Erstellen des Patientenbilddatensatzes nicht bewegt wurde und die Position und Ausrichtung der Ausgabeeinrichtung ebenfalls bekannt ist, kann der Patientenbilddatensatz im Blickfeld des Benutzers deckungsgleich dem realen Patientenkörperteil überlagert werden. Deckungsgleich soll in diesem Fall bedeuten, dass sowohl die Lage und die Ausrichtung als auch die Bildgröße der im Patientenbilddatensatz gespeicherten Bildinformationen im Blickfeld des Chirurgen mit denen des realen Patienten bzw. Patientenkörperteils übereinstimmt. So sieht der Chirurg etwa das durch das Kontrastmittel markierte und durch hell leuchtende Bereiche angedeutete Tumorgewebe an genau der Stelle am realen Patienten bzw. Patientenkörperteil, an welcher es sich auch am oder im realen Patienten bzw. Patientenkörperteil befindet. Falls das Tumorgewebe entfernt oder behandelt werden soll, so muss der Chirurg lediglich das entsprechende medizinische Instrument zum durch das Head-Up-Display eingeblendeten Bereich am oder im Patientenkörperteil führen.

Gemäß einer weiteren Ausführungsform der vorliegenden Erfindung ist es ebenfalls möglich, den Patientenbilddatensatz zusammen mit zumindest einem weiteren Patientenbilddatensatz darzustellen. So ist es denkbar, dass einem schon bestehenden Patientenbilddatensatz, wie etwa einem Ultraschall-, MRT-, MRA- oder CT-Bilddatensatz der durch das erfindungsgemäße Verfahren erstellte Patientenbilddatensatz mit den markierten Bereichen überlagert wird oder parallel zu diesem dargestellt wird. Auch eine Korrelation von dreidimensionalen Datensätzen oder ein MPR-Verfahren ist in diesem Zusammenhang vorstellbar. So ist es denkbar, dass ein durch ICG markiertes Blutgefäß über einen MRA-Bilddatensatz gelegt wird, um die Position des markierten Blutgefäßes in der MRA-Umgebung zu sehen. Dies erlaubt ferner auch eine Beurteilung des sogenannten Brainshifts oder ganz allgemein von Positions- und Lageveränderungen des Gewebes während eines Eingriffs.

Gemäß der Erfindung wurden die Erfassungseinrichtung und die Ausgabeeinrichtung mittels eines medizintechnischen Navigationssystems erfasst, so dass deren Position und Ausrichtung im Raum bekannt ist. Da sowohl die Erfassungseinrichtung als auch die Ausgabeeinrichtung erfasst werden, ist die Darstellung der durch die Erfassungseinrichtung erfassten Bilddaten in räumlich richtiger Position und Ausrichtung zum realen Patienten bzw. Patientenkörperteil möglich, wie weiter oben schon beschrieben wurde. Falls der Patient bzw. Patientenkörperteil ebenfalls registriert wird, d.h. seine Position und Ausrichtung im Raum beispielsweise mittels einer Navigationsreferenz und eines medizintechnischen Navigationssystems getrackt wird, ist eine korrekte Überlagerung des Patientenbilddatensatzes über den realen Patienten auch bei intraoperativen Bewegungen des Patienten bzw. Patientenkörperteils möglich. Ganz allgemein könnte der Patient bzw.

Patientenkörperteil nach dem Erfassen der vom Kontrastmittel ausgehenden Strahlung bewegt werden, ohne die korrekte Überlagerung des Patientenbilddatensatzes über den Patienten bzw. Patientenkörperteil zu gefährden.

Ferner ist es vorstellbar, dass der Patientenkörperteil mittels einer Beleuchtungseinrichtung beleuchtet wird. So können im Bereich des Patientenkörperteils, von dem ein Patientenbilddatensatz erstellt werden soll, Beleuchtungsbedingungen hergestellt werden, welche besonders gute und kontrastreiche Bilder ermöglichen. So ist es insbesondere denkbar, dass das Kontrastmittel durch die Beleuchtungseinrichtung mit UV-Licht, IR-Licht oder sichtbarem Licht bestrahlt wird, um das Kontrastmittel zur Strahlungsemission anzuregen.

Grundsätzlich sind in Verbindung mit der vorliegenden Erfindung vielerlei Kontrastmittel einsetzbar, so beispielsweise strahlungsemittierende Kontrastmittel oder auch eine magnetische Substanz umfassende Kontrastmittel. Die strahlungsemittierenden Kontrastmittel umfassen dabei insbesondere Kontrastmittel, welche Röntgenstrahlung oder Licht emittieren. Im Bereich der lichtemittierenden Kontrastmittel ist ferner die Emission von sichtbarem, Infrarot- oder ultraviolettem Licht denkbar. Bevorzugterweise werden in Verbindung mit der vorliegenden Erfindung Kontrastmittel eingesetzt, welche Fluoreszenzstrahlung oder Phosphoreszenzstrahlung emittieren.

Des Weiteren ist ein Verfahren denkbar, bei dem die Intensität der von den mit dem Kontrastmittel markierten Bereichen emittierten Strahlung während des zweiten Schrittes, also der Darstellung des Patientenbilddatensatzes geringer ist als die Intensität während des ersten Schrittes, also der Erfassung der durch das Kontrastmittel emittierten Strahlung und der Erstellung des Patientenbilddatensatzes auf Basis der erfassten Strahlung. Die Tatsache, dass der auf Basis der erfassten Strahlung erstellte Patientenbilddatensatz beliebig lange speicherbar und zu einem beliebigen gewünschten Zeitpunkt wieder darstellbar ist, erlaubt die Darstellung sehr guter und kontrastreicher Bilder, auch wenn die Strahlungsintensität des Kontrastmittels mittlerweile schon längst abgenommen hat oder das Kontrastmittel nicht mehr an den zu markierenden Bereichen am Patientenkörperteil anhaftet.

Auch wenn die Intensität der von den markierten Bereichen emittierten Strahlung während des zweiten Schrittes geringer ist als die Intensität anderer optischer Strahlung, die vom erfassten Patientenkörperteil ausgeht, können wie oben beschrieben sehr gute und kontrastreiche Bilder dargestellt werden, in denen die durch das Kontrastmittel markierten Bereiche gezeigt werden. Auch wenn während des zweiten Schrittes die Operationssaalbeleuchtung bereits wieder eingeschaltet und die vom Kontrastmittel emittierte Strahlung daher kaum auszumachen ist, kann der Chirurg durch die Darstellung des Patientenbilddatensatzes die durch das Kontrastmittel markierten Bereiche deutlich erkennen und muss nicht wie bisher die Operationssaalbeleuchtung ausschalten, um die vom Kontrastmittel emittierte Strahlung zu sehen.

Die vorliegende Erfindung umfasst ferner eine Vorrichtung zur Darstellung von Bilddaten eines Patientenkörperteils mit einer zumindest eine Kamera umfassenden Erfassungseinrichtung zum Erfassen von Strahlung, die von mit einem strahlungsemittierenden Kontrastmittel versehenen Bereichen des Patientenkörperteils ausgeht, einer Rechnereinheit, die einen Patientenbilddatensatz auf Basis der von der Erfassungseinrichtung erfassten Bilder erstellt und einer Ausgabeeinrichtung zum Darstellen des erstellten Patientenbilddatensatzes.

In einer bevorzugten Ausführungsform erfasst die Erfassungseinrichtung Strahlung im sichtbaren Lichtspektrum. Es ist denkbar, dass die Erfassungseinrichtung Bilder vom Patientenkörperteil bei ausgeschalteter Operationssaalbeleuchtung erfasst, wobei die vom Kontrastmittel emittierte Strahlung die zu markierenden Bereiche besonders gut und kontrastreich vom übrigen Gewebe abhebt. Auch sind Videokameras, Infrarotkameras, Röntgenstrahlendetektoren in Verbindung mit der Erfassungseinrichtung denkbar.

In einer bevorzugten Ausführungsform weist die erfindungsgemäße Vorrichtung eine Erfassungseinrichtung mit zumindest zwei Kameras auf, welche den Patientenkörperteil und insbesondere dessen Oberfläche aus unterschiedlichen Blickrichtungen erfassen. Auf diese Weise können auf mehreren und aus unterschiedlichen Blickrichtungen aufgenommenen Bildern erkennbare Merkmale einander zugeordnet werden. Sofern die Position und Ausrichtung der Kameras im Raum bekannt ist, kann darüber hinaus mittels Triangulation auf die Position dieser Merkmale im Raum geschlossen werden. So ermöglicht eine Erfassungseinrichtung mit zumindest zwei Kameras die Erstellung eines 3D-Patientenbilddatensatzes.

Ferner ist es möglich, dass die Erfassungseinrichtung eine Vielzahl von Bildern zu unterschiedlichen Zeitpunkten erfasst, wobei insbesondere ein Videofilm vom Patientenkörperteil erstellt wird. So können mittels einer Ausgabeeinrichtung dem Chirurgen zu einem späteren Zeitpunkt bewegte Bilddaten vom Patientenkörperteil dargestellt werden und insbesondere in sein Blickfeld eingeblendet werden.

Ferner wird die Erfassungseinrichtung und die Ausgabeeinrichtung von einem medizintechnischen Trackingsystem erfasst. Insbesondere kann die Erfassungseinrichtung und/oder die Ausgabeeinrichtung eine Navigationsreferenz aufweisen, die von einem medizintechnischen Trackingsystem erfasst wird. Auf diese Weise kann die Position und die Ausrichtung der Erfassungseinrichtung und/oder der Ausgabeeinrichtung bestimmt werden, was beispielsweise eine exakte Überlagerung der von der Erfassungseinrichtung erfassten Bilddaten über den realen Patienten bzw. Patientenkörperteil ermöglicht.

Ferner kann die erfindungsgemäße Vorrichtung eine Beleuchtungseinrichtung umfassen, welche die Oberfläche des Patientenkörperteils beleuchtet und insbesondere das Kontrastmittel zur Emission anregt. So kann die Beleuchtungssituation dem jeweiligen Bedürfnis der Erfassungseinrichtung angepasst werden, so dass möglichst scharfe und kontrastreiche Bilder vom Patientenkörperteil erstellt werden. Insbesondere kann durch die Beleuchtung mittels der Beleuchtungseinrichtung das am zu erfassenden Patientenkörperteil befindliche Kontrastmittel zur Emission von Strahlung angeregt werden, wie etwa durch Bestrahlung mit UV-Strahlung oder IR-Strahlung.

Ferner ist es vorstellbar, dass die Rechnereinheit der erfindungsgemäßen Vorrichtung die von der Erfassungseinrichtung erfassten Bilddaten empfängt, auf Basis der erfassten Bilddaten einen Patientenbilddatensatz, insbesondere einen Oberflächenbilddatensatz des Patientenkörperteils erstellt und den Patientenbilddatensatz insbesondere zu einem späteren Zeitpunkt der Ausgabeeinrichtung zur Darstellung bereitstellt. Ferner kann die Rechnereinheit den erstellten Patientenbilddatensatz speichern oder zumindest bis zum Zeitpunkt der Darstellung zwischenspeichern. So können dem Chirurgen zu einem beliebigen späteren Zeitpunkt die von der Erfassungseinrichtung erfassten und von der Rechnereinheit zu einem Patientenbilddatensatz verarbeiteten Bilddaten dargestellt werden. Selbst wenn die Strahlungsintensität des Kontrastmittels in der Zwischenzeit abgenommen hat oder das Kontrastmittel nicht mehr an den zu markierenden Bereichen anhaftet, steht dem Chirurgen ein kontrastreiches Bild des Patientenkörperteils mit den markierten Bereichen zur Verfügung. Gleiches gilt für den Fall, wenn die Operationssaalbeleuchtung in der Zwischenzeit wieder eingeschaltet wurde, so dass die vom Kontrastmittel emittierte Strahlung nicht mehr zu erkennen ist.

Auch ist es in Verbindung mit einer weiteren Ausführungsform möglich, dass die Rechnereinheit die von der Erfassungseinrichtung erfassten Bilddaten zumindest einem weiteren bestehenden Bilddatensatz desselben Patientenkörperteils überlagert. Mit anderen Worten erstellt die Rechnereinheit ein zusammengesetztes Bild aus den mittels der Erfassungseinrichtung erfassten Bilddaten und einem schon bestehenden Patientenbilddatensatz, beispielsweise einem Ultraschall-, einem MRT-, einem MRA- oder einem CT-Bilddatensatz.

Ferner kann die erfindungsgemäße Vorrichtung als Ausgabeeinrichtung eine optische Beobachtungseinrichtung umfassen, was insbesondere ein medizintechnisches Mikroskop sein kann, welches im Speziellen die von der Erfassungseinrichtung erfassten Bilddaten bzw. den Patientenbilddatensatz in das Blickfeld eines Benutzers projiziert. Das medizintechnische Mikroskop kann ein Head-Up-Display umfassen, welches die von der Erfassungseinrichtung erfassten und zu einem Patientenbilddatensatz verarbeiteten Bilder dem durch die optische Beobachtungseinrichtung bzw. das medizintechnische Mikroskop bereitgestellten Bild vom Patientenkörperteil überlagert. Der durch die optische Beobachtungseinrichtung bzw. das medizintechnische Mikroskop blickende Chirurg sieht also die durch das Kontrastmittel hervorgehobenen Bereiche des Patientenkörperteils eingebettet in den momentanen realen durch die optische Beobachtungseinrichtung bzw. das medizintechnische Mikroskop zu sehenden Patientenkörperteil. Vorteilhafterweise muss der Chirurg zum Betrachten der durch das Kontrastmittel markierten Bereiche seinen Blick nicht mehr vom zu behandelnden Patienten bzw. Patientenkörperteil abwenden.

Es ist jedoch auch möglich, dass eine Ausgabeeinrichtung in Form eines Bildschirms oder eines Computermonitors bereitgestellt wird.

Ebenfalls ist es vorstellbar, dass durch andere bildgebende Verfahren erstellte Bilddatensätze parallel oder über die von der Erfassungseinrichtung erfassten Bilddaten überlagert dargestellt werden.

Auch ist ein Projektor als Ausgabeeinrichtung vorstellbar, der den Patientenbilddatensatz auf eine Projektionsfläche oder die Oberfläche des Patientenkörperteils projiziert. Somit werden die durch das Kontrastmittel hervorzuhebenden Bereiche direkt auf die Oberfläche des Patientenkörpers projiziert, so dass der behandelnde Chirurg zum Betrachten der durch das Kontrastmittel hervorzuhebenden Bereiche, beispielsweise Tumorgewebe nicht vom Operationsort aufblicken muss.

Ferner kann das Kontrastmittel ein flüssiges Kontrastmittel sein, welches im Patientenkörperteil oder auf dessen Oberfläche angeordnet ist. Dabei ist es möglich, das flüssige Kontrastmittel mittels einer Injektion oder einer Infusion dem betreffenden Patientenkörperteil zuzuführen, wobei sich das Kontrastmittel bevorzugt an hervorzuhebenden Bereichen, beispielsweise Tumorgewebe anlagert. Sofern das Kontrastmittel Strahlung emittiert, können diese Bereiche anhand der erhöhten Strahlungsintensität vom umliegenden Gewebe unterschieden werden. Auch ist ein manueller Auftrag des Kontrastmittels auf die Oberfläche des Patientenkörperteils denkbar.

Ferner können in Verbindung mit der erfindungsgemäßen Vorrichtung am Patientenkörperteil Marker angeordnet sein, welche insbesondere Infrarotlicht emittierende oder reflektierende Marker sein können, welche von der Erfassungseinrichtung erfasst werden. Dies ist insbesondere dann von Vorteil, wenn in Verbindung mit der erfindungsgemäßen Vorrichtung ein medizintechnisches Trackingsystem Verwendung findet und der Patient bzw. der Patientenkörperteil positionell geortet und registriert werden soll. So ist es in diesem Falle möglich, den Patienten nach der Erfassung der vom Kontrastmittel emittierten Strahlung und der Erstellung eines darauf basierenden Patientenbilddatensatzes zu bewegen, wobei der erstellte Patientenbilddatensatz dem realen Patienten bzw. Patientenkörperteil deckungsgleich überlagert werden kann, sofern Erfassungseinrichtung und Ausgabeeinrichtung ebenfalls durch ein medizintechnisches Trackingsystem in ihrer Lage und Ausrichtung erfasst werden können.

Auch können diese Marker dazu dienen, den erstellten Patientenbilddatensatz in räumlich korrekter Weise dem Patientenkörperteil zu überlagern. So kann etwa direkt von der Ausgabeeinrichtung aus der Abstand zum Patientenkörperteil anhand des Abstandes zwischen den Markern bestimmt werden. Auf diese Weise lässt sich auch der Fokus des Mikroskops automatisch einstellen.

Eine Ausführungsform der vorliegenden Erfindung wird anhand der beiliegenden Figuren näher beschrieben. Die vorliegende Erfindung kann alle hierin beschriebenen Merkmale einzeln sowie in jedweder sinnvollen Kombination umfassen. Es zeigen die:
- Figur 1: eine Ausführungsform der erfindungsgemäßen Vorrichtung, und
- Figur 2: eine Ausführungsform des erfindungsgemäßen Verfahrens.

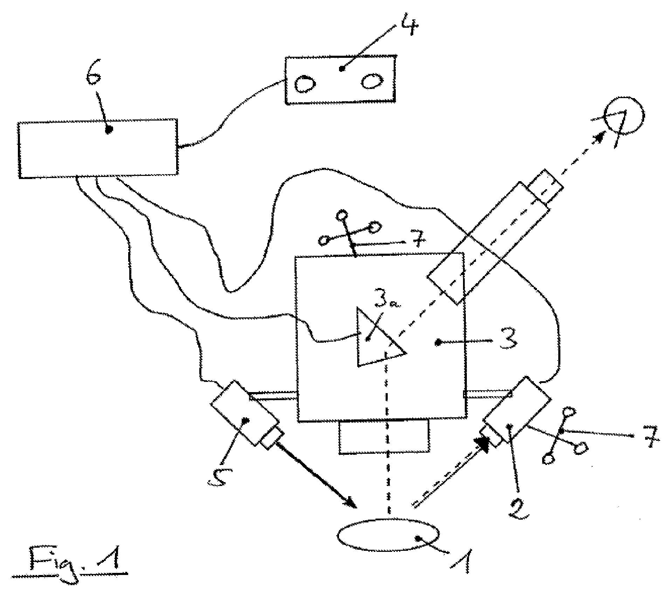

In der Figur 1 ist eine erfindungsgemäße Vorrichtung zur Darstellung von Bilddaten eines Patientenkörperteils gezeigt. Die erfindungsgemäße Vorrichtung umfasst zunächst eine Ausgabeeinrichtung 3 in Form eines medizintechnischen Mikroskops, durch das der Chirurg zum Betrachten eines Patientenkörperteils 1 blickt. Der vom Patientenkörperteil 1 ausgehende und zum Auge des Chirurgen führende optische Strahlengang sei in der Figur 1 durch den gestrichelten Pfeil dargestellt.

Das medizintechnische Mikroskop 3 umfasst ferner eine Navigationsreferenz 7, so dass die Position und Ausrichtung des medizintechnischen Mikroskops 3 im Raum durch das Trackingsystem 4 bestimmt werden kann. Das Trackingsystem 4 umfasst dabei zwei schematisch dargestellte Infrarotkameras, welche die Navigationsreferenz 7 erfassen, so dass mittels Triangulation die Position und Ausrichtung des medizintechnischen Mikroskops 3 mittels des Trackingsystems 4 und der Navigationsreferenz 7 bestimmbar ist. Mit dem medizintechnischen Mikroskop 3 fest verbunden ist eine Beleuchtungseinrichtung 5, welche bei Bedarf den zu erfassenden Patientenkörperteil 1 beleuchtet und insbesondere das am Patientenkörperteil 1 befindliche Kontrastmittel zur Strahlungsemission anregt. Die Beleuchtungsstrahlung sei in der Figur mit dem durchgezogenen Pfeil dargestellt.

Ferner ist mit dem medizintechnischen Mikroskop 3 eine Erfassungseinrichtung 2 verbunden, welche die vom Kontrastmittel am oder im Patientenkörperteil 1 emittierte Strahlung erfasst. Diese emittierte und erfasste Strahlung sei in Figur 1 durch den teils gestrichelten, teils durchgezogenen Pfeil dargestellt. Bei der in der Figur 1 dargestellten Ausführungsform weist auch die Erfassungseinrichtung 2 eine Navigationsreferenz 7 auf, mittels welcher die Position und Ausrichtung der Erfassungseinrichtung 2 im Raum bestimmbar ist. Sofern die Erfassungseinrichtung 2 fest mit dem schon eine Navigationsreferenz 7 aufweisenden Mikroskop 3 verbunden ist, ist eine solche Navigationsreferenz 7 an der Erfassungseinrichtung 2 eigentlich nicht notwendig. Sobald die Erfassungseinrichtung 2 jedoch relativ zum medizintechnischen Mikroskop 3 im Raum bewegbar ist, ist zur Bestimmung der Position und Ausrichtung der Erfassungseinrichtung 2 eine eigene Navigationsreferenz 7 notwendig.

Die Erfassungseinrichtung 2, das medizintechnische Mikroskop 3, das Navigationssystem 4 und die Beleuchtungseinrichtung 5 sind jeweils über Datenleitungen mit einer Rechnereinheit 6 verbunden. Diese Datenleitungen können sowohl kabelgestützte als auch funkgestützte Datenleitungen sein.

Zum Aufnehmen eines Bildes vom Patientenkörperteil 1 wird beispielsweise die Operationssaalbeleuchtung ausgeschaltet und dafür bei Bedarf die Beleuchtungseinrichtung 5 eingeschaltet. Die vom Kontrastmittel am oder im Patientenkörperteil 1 emittierte Strahlung wird von der Erfassungseinrichtung 2 erfasst und an die Rechnereinheit 6 weitergegeben. Auf Basis dieser Bildinformationen wird von der Rechnereinheit 6 ein Patientenbilddatensatz erstellt, welcher über das im Zentrum des medizintechnischen Mikroskops 3 angeordneten Head-Up-Displays 3a dargestellt wird.

Blickt nun ein Chirurg durch das medizintechnische Mikroskop 3, um den Patientenkörperteil 1 zu betrachten, kann der erstellte Patientenbilddatensatz mit den durch das Kontrastmittel markierten Bereichen mittels des Head-Up-Displays 3a in das Blickfeld des Chirurgen eingeblendet werden. Da sowohl die Erfassungseinrichtung 2 als auch das medizintechnische Mikroskop 3 jeweils durch Navigationsreferenzen 7 vom medizintechnischen Trackingsystem 4 in ihrer Position und Ausrichtung erfassbar sind, ist es der Rechnereinheit 6 auch möglich, den erstellten Patientenbilddatensatz mittels des Head-Up-Displays 3a so darzustellen, dass der Chirurg den erstellten Patientenbilddatensatz immer deckungsgleich zum realen Patientenkörperteil 1 sieht. Um etwa ein durch Kontrastmittel markiertes Tumorgewebe zu entfernen, muss er lediglich den Bereich des Patientenkörperteils 1 entfernen, welcher durch den durch das Kontrastmittel markierten und mittels des Head-Up-Displays 3a eingeblendeten Bereich überlagert wird.

Auch kann im Anschluss daran erneut ein Patientenbilddatensatz mit dem verbleibenden Tumorgewebe erstellt werden, um den Operationserfolg zu beurteilen. Gegebenenfalls kann das erfindungsgemäße Verfahren wiederholt werden, um das verbleibende Tumorgewebe zu entfernen.

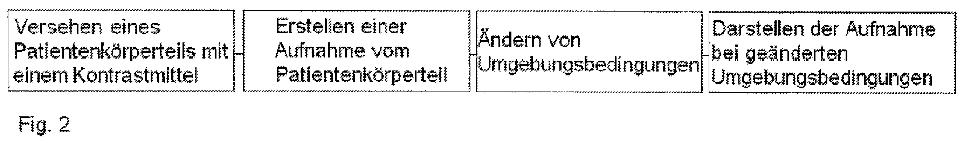

In der Figur 2 ist eine prinzipielle Ausführungsform des erfindungsgemäßen Verfahrens zu sehen.

Dabei wird zunächst ein Patientenkörperteil mit einem Kontrastmittel versetzt, beispielsweise ALA zur Markierung von Tumorgewebe oder ICG zur Markierung von Blutgefäßen. Im Anschluss daran wird von diesem Patientenkörperteil mittels einer Erfassungseinrichtung eine Aufnahme erstellt, wobei auf den Aufnahmen die durch das Kontrastmittel markierten Bereiche anhand der höheren Strahlungsintensität vom übrigen Gewebe des Patientenkörperteils differenziert werden können. Zum Erstellen solcher Aufnahmen sind zumeist spezielle Umgebungsbedingungen notwendig, beispielsweise eine in ihrer Helligkeit deutlich reduzierte oder gar ganz ausgeschaltete Operationssaalbeleuchtung. Nur bei solchen Beleuchtungsverhältnissen lässt sich die schwache vom Kontrastmittel emittierte Strahlung ausmachen und ein gutes und kontrastreiches Bild vom mit dem Kontrastmittel versetzten Patientenkörperteil erstellen. Sobald die Bilder bzw. ein Videofilm vom Patientenkörperteil unter den speziellen Umgebungsbedingungen erstellt wurden, können in einem nächsten Schritt die Umgebungsbedingungen den normalen Verhältnissen angepasst werden. Es kann nach dem Erstellen der Bilder die Operationssaalbeleuchtung in ihrer Intensität wieder so eingestellt werden, dass ein normaler Operationsablauf möglich ist.

Da zu diesem Zeitpunkt, also der normalen Operationssaalbeleuchtung die vom Kontrastmittel emittierte Strahlung nicht mehr auszumachen ist, werden in einem nächsten Schritt die erstellten Aufnahmen bzw. der erstellte Patientenbilddatensatz mittels der Ausgabeeinrichtung dargestellt.

## Patentansprüche

1. Verfahren zur Darstellung von Bilddaten eines Patientenkörperteils, wobei der Patientenkörperteil (1) mit Bereichen, die zuvor mit Strahlung emittierendem Kontrastmittel versehen wurden, in einem ersten Schritt mittels einer Erfassungseinrichtung (2) erfasst wird, wobei während des ersten Schritts die Intensität der von den mit dem Kontrastmittel versehenen Bereichen ausgehenden Strahlung höher ist als die Intensität anderer von der Erfassungseinrichtung (2) erfassbarer Strahlung, die vom erfassten Patientenkörperteil (1) ausgeht, wobei auf Basis der erfassten Strahlung ein Patientenbilddatensatz erstellt und gespeichert wird, **dadurch gekennzeichnet, dass** die Position und Ausrichtung der Erfassungseinrichtung (2) und einer Ausgabeeinrichtung (3) relativ zum Patientenkörperteil mittels eines medizintechnischen Trackingsystems (4) erfasst wird, und der Patientenbilddatensatz in einem zweiten Schritt in Abhängigkeit der relativen Position und Ausrichtung von Ausgabeeinrichtung (3) und Patientenkörperteil (1) dem Blickfeld eines Benutzers mittels der Ausgabeeinrichtung (3) zu einem beliebigen späteren Zeitpunkt deckungsgleich zum realen Patientenkörperteil (1) überlagert wird.

2. Verfahren nach Anspruch 1, wobei das Kontrastmittel Strahlung im sichtbaren Lichtspektrum emittiert.

3. Verfahren nach einem der Ansprüche 1 oder 2, wobei der Patientenbilddatensatz die Oberflächendaten des Patientenkörperteils (1) umfasst.

4. Verfahren nach einem der Ansprüche 1 bis 3, wobei der Patientenbilddatensatz eine Vielzahl von zu unterschiedlichen Zeitpunkten erfassten Bildern vom Patientenkörperteil (1), insbesondere eine Videoaufnahme vom Patientenkörperteil (1) umfasst.

5. Verfahren nach einem der Ansprüche 1 bis 4, wobei der Patientenkörperteil (1) für die Erstellung eines 3D-Patientenbilddatensatzes im ersten Schritt mittels der Erfassungseinrichtung (2) aus zumindest zwei unterschiedlichen Blickrichtungen erfasst wird.

6. Verfahren nach einem der Ansprüche 1 bis 5, wobei der Patientenbilddatensatz im zweiten Schritt mittels der Ausgabeeinrichtung (3) zusammen mit zumindest einem weiteren Patientenbilddatensatz, insbesondere zumindest einem weiteren Patientenbilddatensatz überlagert dargestellt wird.

7. Verfahren nach einem der Ansprüche 1 bis 6, wobei der Patientenkörperteil (1) mittels einer Beleuchtungseinrichtung (5) beleuchtet und insbesondere das Kontrastmittel zur Strahlungsemission angeregt wird.

8. Verfahren nach einem der Ansprüche 1 bis 7, wobei die vom Kontrastmittel emittierte Strahlung Fluoreszenzstrahlung oder Lumineszenzstrahlung ist.

9. Verfahren nach einem der Ansprüche 1 bis 8, wobei die Intensität der von den mit dem Kontrastmittel versetzten Bereichen emittierten Strahlung während des zweiten Schrittes geringer ist als die Intensität während des ersten Schrittes.

10. Verfahren nach einem der Ansprüche 1 bis 9, wobei die Intensität der von den mit dem Kontrastmittel versetzten Bereichen emittierten Strahlung während des zweiten Schrittes geringer ist als die Intensität anderer optischer Strahlung, die vom erfassten Patientenkörperteil (1) ausgeht.

11. Vorrichtung zur Darstellung von Bilddaten eines Patientenkörperteils mit
- einer zumindest eine Kamera (2) umfassenden und in ihrer Position und Ausrichtung relativ zum Patientenkörperteil (1) mittels eines medizintechnischen Trackingsystems (4) erfassbaren Erfassungseinrichtung (2) zum Erfassen von Strahlung, die von mit einem Strahlung emittierenden Kontrastmittel versehenen Bereichen des Patientenkörperteils (1) ausgeht,
- einer Rechnereinheit (6), die einen Patientenbilddatensatz auf Basis der von der Erfassungseinrichtung (2) erfassten Bilder erstellt und diesen speichert, und
- einer in ihrer Position und Ausrichtung relativ zum Patientenkörperteil (1) mittels des medizintechnischen Trackingsystems (4) erfassbaren Ausgabeeinrichtung (3), die dem Blickfeld eines Benutzers in Abhängigkeit der relativen Position und Ausrichtung von Ausgabeeinrichtung (3) und Patientenkörperteil (1) den erstellten Patientenbilddatensatz zu einem beliebigen späteren Zeitpunkt und deckungsgleich zum realen Patientenkörperteil (1) überlagert.

12. Vorrichtung nach Anspruch 11, wobei Strahlung im sichtbaren Lichtspektrum emittiert und von der Erfassungseinrichtung (2) erfasst wird.

13. Vorrichtung nach einem der Ansprüche 11 oder 12, wobei die Erfassungseinrichtung (2) zumindest zwei Kameras (2) umfasst, welche den Patientenkörperteil (1), insbesondere dessen Oberfläche aus unterschiedlichen Blickrichtungen erfassen.

14. Vorrichtung nach einem der Ansprüche 11 bis 13, wobei die Erfassungseinrichtung (2) eine Vielzahl von Bildern zu unterschiedlichen Zeitpunkten erfasst, insbesondere ein Video vom Patientenkörperteil (1) erfasst.

15. Vorrichtung nach einem der Ansprüche 11 bis 14, mit ferner einer Beleuchtungseinrichtung (5), welche die Oberfläche des Patientenkörperteils (1) beleuchtet und insbesondere das Kontrastmittel zur Emission anregt.

16. Vorrichtung nach einem der Ansprüche 11 bis 15, wobei die Rechnereinheit (6) die von der Erfassungseinrichtung (2) erfassten Bilddaten empfängt, auf Basis der erfassten Bilddaten einen Patientenbilddatensatz, insbesondere einen Oberflächenbilddatensatz des Patientenkörperteils (1) erstellt und den Patientenbilddatensatz insbesondere zu einem späteren Zeitpunkt der Ausgabeeinrichtung (3) zur Darstellung bereitstellt.

17. Vorrichtung nach einem der Ansprüche 11 bis 16, wobei die Rechnereinheit (6) die von der Erfassungseinrichtung (2) erfassten Bilddaten zumindest einem weiteren bestehenden Bilddatensatz desselben Patientenkörperteils (1) überlagert.

18. Vorrichtung nach einem der Ansprüche 11 bis 17, wobei die Ausgabeeinrichtung (3) eine optische Beobachtungseinrichtung, insbesondere ein medizintechnisches Mikroskop ist, welches im Speziellen die von der Erfassungseinrichtung (2) erfassten Bilddaten in das Blickfeld eines Benutzers projiziert.

19. Vorrichtung nach einem der Ansprüche 11 bis 18, wobei die Ausgabeeinrichtung (3) ein Bildschirm ist.

20. Vorrichtung nach einem der Ansprüche 11 bis 18, wobei die Ausgabeeinrichtung (3) ein Projektor ist, der den Patientenbilddatensatz auf die Oberfläche des Patientenkörperteils (1) projiziert.

21. Vorrichtung nach einem der Ansprüche 11 bis 20, wobei das Kontrastmittel ein flüssiges Kontrastmittel ist, welches im Patientenkörperteil (1) oder auf dessen Oberfläche angeordnet ist.

22. Vorrichtung nach einem der Ansprüche 11 bis 21, wobei die Erfassungseinrichtung (2) am Patientenkörperteil (1) angeordnete Marker, insbesondere Infrarotlicht emittierende oder reflektierende Marker erfasst.

## Claims

1. A method for displaying image data of a part of a patient's body, wherein the part of the patient's body (1) comprising regions which have been provided with a radiation-emitting contrast agent beforehand is detected in a first step by means of a detection means (2), wherein during the first step, the intensity of the radiation emitted by the regions provided with the contrast agent is higher than the intensity of other radiation which is emitted by the detected part of the patient's body (1) and which can be detected by the detection means (2), wherein a patient image dataset is produced on the basis of the detected radiation and stored, **characterised in that** the position and alignment of the detection means (2) and of an output means (3) relative to the part of the patient's body is detected by means of a medical tracking system (4), and the patient image dataset is superimposed onto a user's field of view in a second step, at any subsequent time and congruent with the actual part of the patient's body (1), by means of the output means (3) and in accordance with the relative position and alignment of the output means (3) and the part of the patient's body (1).

2. The method according to Claim 1, wherein the contrast agent emits radiation in the visible light spectrum.

3. The method according to any one of Claims 1 or 2, wherein the patient image dataset includes the surface data of the part of the patient's body (1).

4. The method according to any one of Claims 1 to 3, wherein the patient image dataset includes a multitude of images of the part of the patient's body (1), captured at different times, in particular a video recording of the part of the patient's body (1).

5. The method according to any one of Claims 1 to 4, wherein the part of the patient's body (1) is detected in the first step by means of the detection means (2) from at least two different viewing directions, in order to produce a 3D patient image dataset.

6. The method according to any one of Claims 1 to 5, wherein the patient image dataset is displayed in the second step by means of the output means (3) together with at least one other patient image dataset, in particular superimposed onto at least one other patient image dataset.

7. The method according to any one of Claims 1 to 6, wherein the part of the patient's body (1) is illuminated by means of an illumination means (5), and the contrast agent is in particular stimulated to emit radiation.

8. The method according to any one of Claims 1 to 7, wherein the radiation emitted by the contrast agent is fluorescent or luminescent radiation.

9. The method according to any one of Claims 1 to 8, wherein the intensity of the radiation emitted by the regions provided with the contrast agent is lower during the second step than the intensity during the first step.

10. The method according to any one of Claims 1 to 9, wherein the intensity of the radiation emitted by the regions provided with the contrast agent is lower during the second step than the intensity of other optical radiation which is emitted by the detected part of the patient's body (1).

11. A device for displaying image data of a part of a patient's body, comprising:
- a detection means (2), which includes at least one camera (2) and the position and alignment of which relative to the part of the patient's body (1) can be detected by means of a medical tracking system (4), for detecting radiation which is emitted by regions of the part of the patient's body (1) provided with a radiation-emitting contrast agent;
- a computer unit (6) which produces a patient image dataset on the basis of the images captured by the detection means (2) and stores said patient image dataset; and
- an output means (3), the position and alignment of which relative to the part of the patient's body (1) can be detected by means of the medical tracking system (4), which superimposes the produced patient image dataset onto a user's field of view, at any subsequent time and congruent with the actual part of the patient's body (1), in accordance with the relative position and alignment of the output means (3) and the part of the patient's body (1).

12. The device according to Claim 11, wherein radiation is emitted in the visible light spectrum and detected by the detection means (2).

13. The device according to any one of Claims 11 or 12, wherein the detection means (2) includes at least two cameras (2) which detect the part of the patient's body (1), in particular its surface, from different viewing directions.

14. The device according to any one of Claims 11 to 13, wherein the detection means (2) captures a multitude of images at different times and in particular captures a video of the part of the patient's body (1).

15. The device according to any one of Claims 11 to 14, further comprising an illumination means (5) which illuminates the surface of the part of the patient's body (1) and in particular stimulates the contrast agent to emit.

16. The device according to any one of Claims 11 to 15, wherein the computer unit (6): receives the image data captured by the detection means (2); produces a patient image dataset, in particular a surface image dataset, of the part of the patient's body (1) on the basis of the captured image data; and provides the patient image dataset to the output means (3), in particular at a subsequent time, for displaying.

17. The device according to any one of Claims 11 to 16, wherein the computer unit (6) superimposes the image data captured by the detection means (2) onto at least one other existing image dataset of the same part of the patient's body (1).

18. The device according to any one of Claims 11 to 17, wherein the output means (3) is an optical observation means, in particular a medical microscope, which specifically projects the image data captured by the detection means (2) into a user's field of view.

19. The device according to any one of Claims 11 to 18, wherein the output means (3) is a screen.

20. The device according to any one of Claims 11 to 18, wherein the output means (3) is a projector which projects the patient image dataset onto the surface of the part of the patient's body (1).

21. The device according to any one of Claims 11 to 20, wherein the contrast agent is a liquid contrast agent which is disposed in the part of the patient's body (1) or on its surface.

22. The device according to any one of Claims 11 to 21, wherein the detection means (2) detects markers, in particular markers which emit or reflect infrared light, which are disposed on the part of the patient's body (1).

## Revendications

1. Procédé pour la représentation de données d'image d'une partie de corps d'un patient, où lors d'une première étape, la partie de corps du patient (1) avec des zones préalablement pourvues d'un produit de contraste émettant un rayonnement est capturée à l'aide de moyens de capture (2), où lors de la première étape, l'intensité du rayonnement émis par les zones pourvues du produit de contraste est plus forte que l'intensité d'autres rayonnements détectables par les moyens de capture (2) émis par la partie de corps du patient (1) capturée, où un jeu de données d'image de patient est constitué et sauvegardé à partir du rayonnement détecté, **caractérisé en ce que** la position et l'orientation des moyens de capture (2) et d'un moyen de sortie (3) par rapport à la partie de corps du patient est détectée à l'aide d'un système de localisation médico-technique (4) et, lors d'une seconde étape, le jeu de données d'images du patient est superposé, à n'importe quel moment ultérieur, à l'aide du moyen de sortie (3), dans le champ de vision d'un utilisateur, sur la partie de corps réelle du patient (1), en fonction de la position et de l'orientation relative du moyen de sortie (3) et de la partie de corps du patient (1).

2. Procédé selon la revendication 1, où le produit de contraste émet un rayonnement dans la partie visible du spectre.

3. Procédé selon l'une des revendications 1 ou 2, où le jeu de données d'image du patient comprend les données de surface de la partie de corps du patient (1).

4. Procédé selon l'une des revendications 1 à 3, où le jeu de données d'image du patient comprend une pluralité d'images de la partie de corps du patient (1) capturées à des moments différents, en particulier un enregistrement vidéo de la partie de corps du patient (1).

5. Procédé selon l'une des revendications 1 à 4, où, pour constituer un jeu de données d'images 3D du patient lors de la première étape, la partie de corps du patient (1) est capturée à l'aide des moyens de capture (2) depuis au moins deux directions d'observation différentes.

6. Procédé selon l'une des revendications 1 à 5, où, lors de la seconde étape, le jeu de données d'image du patient est représenté à l'aide du moyen de sortie (3) en superposition avec au moins un autre jeu de données d'image du patient, en particulier avec au moins un autre jeu de données d'image du patient.

7. Procédé selon l'une des revendications 1 à 6, où la partie de corps du patient (1) est éclairée à l'aide d'un moyen d'éclairage (5) et le produit de contraste, en particulier, est excité de manière à ce qu'il émette un rayonnement.

8. Procédé selon l'une des revendications 1 à 7, où le rayonnement émis par le produit de contraste est un rayonnement fluorescent ou un rayonnement luminescent.

9. Procédé selon l'une des revendications 1 à 8, où l'intensité du rayonnement émis par les zones pourvues du produit de contraste est plus faible durant la seconde étape que durant la première étape.

10. Procédé selon l'une des revendications 1 à 9, où l'intensité du rayonnement émis par les zones pourvues du produit de contraste durant la seconde étape est plus faible que l'intensité d'autres rayonnements optiques émis par la partie de corps du patient (1) capturée.

11. Dispositif pour la représentation de données d'image d'une partie de corps d'un patient avec
- des moyens de capture (2), comprenant au moins une caméra (2) et dont la position et l'orientation par rapport à la partie de corps du patient (1) est détectable au moyen d'un système de localisation médico-technique, pour capturer un rayonnement émis par des zones de la partie de corps du patient (1) pourvues d'un produit de contraste émettant un rayonnement,
- une unité de calcul (6) qui constitue un jeu de données d'image du patient sur la base des images capturées par les moyens de capture (2) et le sauvegarde, et
- un moyen de sortie (3) dont la position et l'orientation par rapport à la partie de corps du patient (1) est détectable au moyen d'un système de localisation médico-technique (4) et qui superpose le jeu de données d'images du patient constitué, à n'importe quel moment ultérieur, dans le champ de vision d'un utilisateur, sur la partie de corps réelle du patient (1) en fonction de la position et de l'orientation relative du moyen de sortie (3) et de la partie de corps du patient (1).

12. Dispositif selon la revendication 11, où un rayonnement dans la partie visible du spectre est émis et capturé par les moyens de capture (2).

13. Dispositif selon la revendication 11 ou 12, où les moyens de capture (2) comprennent au moins deux caméras (2) qui capturent la partie de corps du patient (1), en particulier sa surface, depuis différentes directions d'observation.

14. Dispositif selon l'une des revendications 11 à 13, où les moyens de capture (2) capturent une pluralité d'images à des moments différents, en particulier une vidéo de la partie de corps du patient (1).

15. Dispositif selon l'une des revendications 11 à 14, avec en outre un moyen d'éclairage (5) qui éclaire la surface de la partie de corps du patient (1) et en particulier excite le produit de contraste pour l'émission d'un rayonnement.

16. Dispositif selon l'une des revendications 11 à 15, où l'unité de calcul (6) reçoit les données d'image capturées par les moyens de capture (2), constitue un jeu de données d'image du patient, en particulier un jeu de données d'image de surface de la partie de corps du patient (1), à partir des données d'image capturées et fournit le jeu de données d'images de patient au moyen de sortie (3) pour sa représentation, en particulier à un moment ultérieur.

17. Dispositif selon l'une des revendications 11 à 16, où l'unité de calcul (6) superpose les données d'image capturées par les moyens de capture (2) sur au moins un autre jeu de données d'image existant de la même partie de corps du patient (1).

18. Dispositif selon l'une des revendications 11 à 17, où le moyen de sortie (3) est un moyen d'observation optique, en particulier un microscope médico-technique, qui, en particulier, projette les données d'image capturées par les moyens de capture (2) dans le champ de vision d'un utilisateur.

19. Dispositif selon l'une des revendications 11 à 18, où le moyen de sortie (3) est un écran.

20. Dispositif selon l'une des revendications 11 à 18, où le moyen de sortie (3) est un projecteur (3) qui projette le jeu de données d'image du patient sur la surface de la partie de corps du patient (1).

21. Dispositif selon l'une des revendications 11 à 20, où le produit de contraste est un produit de contraste liquide qui est disposé dans la partie de corps de patient (1) ou sur sa surface.

22. Dispositif selon l'une des revendications 11 à 21, où les moyens de capture (2) capturent des marqueurs disposés sur la partie de corps de patient (1), en particulier des marqueurs émettant ou réfléchissant de la lumière infrarouge.
